(19) Europäisches Patentamt
European Patent Office
Office européen des brevets

(11) Veröffentlichungsnummer : **0 044 497**
**B1**

# (12) EUROPÄISCHE PATENTSCHRIFT

(45) Veröffentlichungstag der Patentschrift : **28.11.84**

(51) Int. Cl.$^3$ : **C 07 D235/26**, C 07 D263/58, C 07 D277/68

(21) Anmeldenummer : **81105457.6**

(22) Anmeldetag : **13.07.81**

## (54) Verfahren zur Herstellung heterocyclisch substituierter 4-Oxiphenoxialkancarbonsäurederivate.

(30) Priorität : **19.07.80 DE 3027483**

(43) Veröffentlichungstag der Anmeldung : **27.01.82 Patentblatt 82/04**

(45) Bekanntmachung des Hinweises auf die Patenterteilung : **28.11.84 Patentblatt 84/48**

(84) Benannte Vertragsstaaten : **BE CH DE FR GB LI NL**

(56) Entgegenhaltungen :
**AT-B- 322 550**
**DE-A- 2 640 730**
**DE-A- 2 758 002**
**DE-A- 2 830 066**
**DE-A- 2 914 300**
**DE-A- 3 018 003**

(73) Patentinhaber : **HOECHST AKTIENGESELLSCHAFT**
**Postfach 80 03 20**
**D-6230 Frankfurt am Main 80 (DE)**

(72) Erfinder : **Willms, Lothar, Dr.**
**Grüner Weg 4**
**D-5463 Unkel (DE)**
Erfinder : **Handte, Reinhard, Dr.**
**Breckenheimer Strasse 45**
**D-6238 Hofheim am Taunus (DE)**
Erfinder : **Mildenberger, Hilmar, Dr.**
**Fasanenstrasse 24**
**D-6233 Kelkheim (Taunus) (DE)**

**EP 0 044 497 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung ist ein Verfahren zur Herstellung von 2-[4'-Benzthiazolyl-2"-oxi-phenoxi] alkancarbonsäurederivaten der allgemeinen Formel I

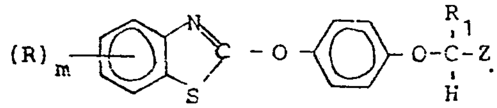

(I)

worin

R Halogen, $CF_3$, $NO_2$, CN, ($C_1$-$C_4$)-Alkyl, ($C_1$-$C_4$)-Alkoxi oder ($C_1$-$C_4$)-Alkylthio,

$R_1$ H oder ($C_1$-$C_4$)-Alkyl,

Z eine Gruppe der Formel

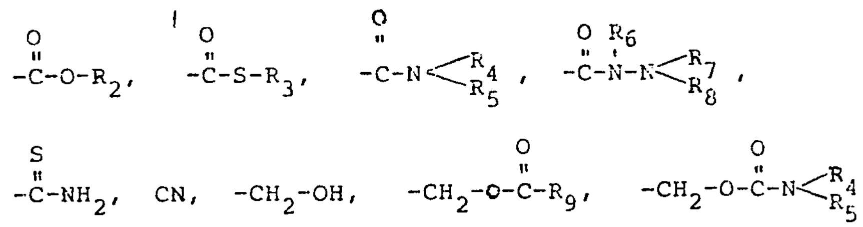

oder $-CH_2-O-SO_2-R_{10}$,

m 0 bis 2

$R_2$ H($C_1$-$C_{12}$)-Alkyl, das gegebenenfalls durch 1-6 Halogen und/oder durch OH, ($C_1$-$C_6$)-Alkoxi, ($C_1$-$C_4$)-Alkylthio, ($C_1$-$C_6$)-Alkoxi-($C_2$-$C_6$)-alkoxi, Halogen ($C_1$-$C_2$)-alkoxi, Methoxi-äthoxi-äthoxi, ($C_1$-$C_4$)-Alkylamino, Di-($C_1$-$C_4$)-alkyl-amino, Phenyl, Oxiranyl und/oder Phenoxi substituiert ist, wobei letzteres ebenfalls ein- bis zweifach durch Halogen oder ($C_1$-$C_4$)-Alkyl substituiert sein kann, ($C_5$-$C_6$)-Cycloalkyl, das gegebenenfalls durch Halogen oder Methyl substituiert ist, ($C_3$-$C_6$)-Alkenyl, Halogen-($C_3$-$C_6$)-alkenyl oder ($C_5$-$C_6$)-Cycloalkenyl, ($C_3$-$C_4$)-Alkinyl, das gegebenenfalls ein- oder zweifach durch ($C_1$-$C_6$)-Alkyl, Phenyl, Halogen bzw. ($C_1$-$C_2$)-Alkoxi substituiert ist, Phenyl, das gegebenenfalls ein- bis dreifach durch ($C_1$-$C_4$)-Alkyl, ($C_1$-$C_4$)-Alkoxi, Halogen, $NO_2$ oder $CF_3$ substituiert ist, Furfuryl, Tetrahydrofurfuryl oder ein Kationäquivalent einer organischen oder anorganischen Base, oder Reste der Formeln

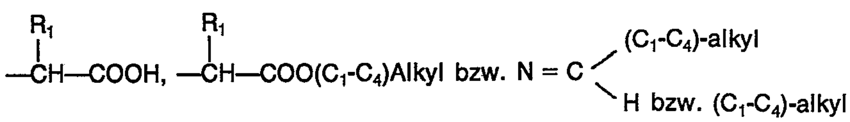

$R_3$ ($C_1$-$C_6$)-Alkyl, Phenyl-($C_1$-$C_2$)-alkyl, wobei der Phenylrest ein- oder zweifach durch ($C_1$-$C_4$)-Alkyl und/oder Halogen substituiert sein kann, ($C_3$-$C_6$)-Alkenyl oder Phenyl, das auch ein- oder zweifach durch ($C_1$-$C_4$)-Alkyl und/oder Halogen substituiert sein kann,

$R_4$ und $R_5$ gleich oder verschieden sind und H, ($C_1$-$C_6$)-Alkyl, Hydroxy-($C_1$-$C_6$)-alkyl, ($C_5$-$C_6$)-Cycloalkyl oder Phenyl, das gegebenenfalls ein- bis dreifach durch ($C_1$-$C_4$)-Alkyl, ($C_1$-$C_4$)-Alkoxi, Halogen oder $CF_3$ substituiert ist, mit der Maßgabe, daß $R_4$ und $R_5$ nicht gemeinsam Phenyl sind, bedeuten oder gemeinsam eine Methylenkette mit 2, 4 oder 5 Gliedern bilden, in der eine $CH_2$-Gruppe gegebenenfalls durch O, NH oder $N(CH_3)$ ersetzt sein kann,

$R_6$ H oder $CH_3$,

$R_7$ H, $CH_3$ oder $C_2H_5$,

$R_8$ H, $CH_3$, $C_2H_5$ oder Phenyl,

$R_9$ ($C_1$-$C_6$)-Alkyl, das gegebenenfalls ein- bis dreifach durch Halogen substituiert ist, Cyclopropyl, ($C_3$-$C_6$)-Alkenyl, Phenyl, ($C_1$-$C_4$)-Alkyl-phenyl, ($C_1$-$C_4$)-Alkoxi-phenyl, Halogen-phenyl, Trifluormethyl-phenyl oder Nitrophenyl sowie,

$R_{10}$ ($C_1$-$C_4$)-Alkyl oder Phenyl, das auch ein- oder zweifach durch Halogen, $CF_3$, $NO_2$ oder ($C_1$-$C_4$)-Alkyl substituiert sein kann,

bedeuten, das dadurch gekennzeichnet ist, daß man Phenole der Formel II

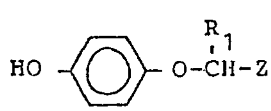

(II)

mit Verbindungen der Formel III

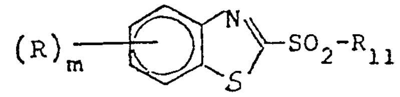

(III)

in der

$R_{11}$ einen niedermolekularen Alkylrest oder einen Phenylrest bedeudet, in Gegenwart basischer Verbindungen umsetzt.

Die Verbindungen der Formel I sind z. B. aus der DE-A 26 40 730 bekannt oder wurden in den deutschen Offenlegungsschriften DE-A 29 14 300.8 und DE-A 30 18 003.1 vorgeschlagen. Sie zeichnen sich durch eine hervorragende herbizide Wirksamkeit insbesondere gegen grasartige Unkräuter aus.

Die Verbindungen der allgemeinen Formel I lassen sich z. B. durch Umsetzung entsprechender Phenole der Formel II mit 2-Halogenbenzthiazolen der allgemeinen Formel IV

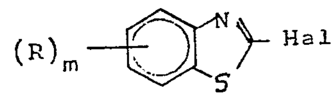

(IV)

(Hal = Cl oder Br)

herstellen. Diese Umsetzung hat jedoch den Nachteil, daß relativ lange Reaktionszeiten erforderlich sind. So wird z. B. bei der Reaktion von 2-(4'-Hydroxiphenoxi)-propionsäureäthylester mit 2-Chlorbenzthiazol und Kaliumcarbonat als Basenzusatz in Acetonitril erst nach 30-stündigem Erhitzen unter Rückfluß eine 88,5 %ige Ausbeute an 2-[4'-(Benzthiazol-2''-yl-oxi)-phenoxi] propionsäureäthylester erhalten. Infolge dieser relativ drastischen Reaktionsbedingungen kommt es zu einer partiellen Verseifung der Estergruppe Z in der Phenolkomponente II bzw. der Endprodukte I unter Ausbildung der entsprechenden Carbonsäuren. Auch bei den anderen Verbindungen der allgemeinen Formeln I und II sind irreversible Veränderungen des Substituenten Z unter den gewählten Reaktionsbedingungen nicht immer auszuschließen.

Nach dem erfindungsgemäßen Verfahren lassen sich prinzipiell alle Verbindungen der allgemeinen Formel I darstellen.

Bevorzugt wird das Verfahren zur Herstellung von Verbindungen der Formel I angewandt, bei denen der Rest

R F, Cl, Br, $CF_3$ oder $NO_2$ in 6-Stellung,
m 0 oder 1,
$R_1$ Methyl und
Z —$COOR_2$ bedeutet.

Bei Verwendung von optisch aktiven 2-(4'-Hydroxyphenoxy)-alkancarbonsäureestern der Formel II als Ausgangsstoffe lassen sich optisch aktive Endprodukte erhalten, wie sie z. B. in der DE-OS 27 58 002 beschrieben sind.

Die Phenole der Formel II sind aus der Literatur bekannt (z. B. J. Org. Chem. *39,* 214 (1974)) oder können in Analogie zu bekannten Verfahren hergestellt werden. Die Verbindungen der Formel III lassen sich nach bekannten Verfahren z. B. aus den entsprechenden 2-Mercaptobenzthiazolen durch Alkylierung und anschließende Oxidation mit Kaliumpermanganat, Wasserstoffperoxid oder organischen Persäuren herstellen (vgl. J. Chem. Soc. *1949,* 3311 ; Eur. J. Med. Chem. *1978*, 171 ; J. Heterocycl. Chem. *13*, 491 (1976) ; Chem. Pharm. Bull. *17*, 1958 (1969)).

Wegen der Bedeutung der Verbindungen I als Pflanzenschutzmittel bestand ein dringendes Bedürfnis nach einem verbesserten Herstellungsverfahren.

Das erfindungsgemäße Verfahren vermeidet die oben beschriebenen Nachteile. Es liefert bereits nach relativ kurzer Reaktionszeit die Verbindungen der Formel I in hohen Ausbeuten und guter Reinheit, ohne daß die im Rest Z vorhandenen funktionellen Gruppen angegriffen werden. Dies war überraschend und aufgrund des Standes der Technik nicht vorhersehbar. Zwar ist die Umsetzung von 2-Alkylsulfonylbenzthiazolderivaten mit aliphatischen Alkoholen, primären oder sekundären Aminen oder CH-aciden Verbindungen bereits bekannt (vgl. J. Chem. Soc. *1949,* 3311 ; J. Heterocycl. Chem. *13*, 491 (1976) ; Eur. J. Med. Chem. *1978*, 171), doch sind entsprechende Umsetzungen mit Phenolen bisher nicht beschrieben worden.

Beispiele für Ausgangsstoffe der Formel III sind ;

2-Methylsulfonyl-benzthiazol,
2-Äthylsulfonyl-benzthiazol,
6-Chlor-2-methylsulfonylbenzthiazol,
6-Brom-2-methylsulfonyl-benzthiazol.

Bei der erfindungsgemäßen Umsetzung wird die nucleofuge Gruppe $—SO_2—R_{11}$ durch die deprotonierte Phenolkomponente ersetzt. Läßt man zum Beispiel 6-Chlor-2-methylsulfonyl-benzthiazol unter Zusatz von Kaliumcarbonat mit einem Phenol der Formel II reagieren, so isoliert man nach der Reaktion neben der gewünschten Verbindung I und Kaliumhydrogencarbonat Kaliummethylsulfinat in fast quantitativer Ausbeute. Das Molverhältnis der Reaktionskomponenten II und III ist nicht kritisch und kann in weiten Grenzen variiert werden. Da jedoch die Abtrennung von nicht umgesetztem Ausgangsprodukt zusätzliche Operationen erfordert, arbeitet man bevorzugt mit etwa äquimolaren Mengen bzw. mit einem Überschuß von nicht mehr als 10 % der Verbindung II.

Das erfindungsgemäße Verfahren kann in An- oder Abwesenheit von Lösungsmitteln durchgeführt werden. Bevorzugt werden die Umsetzungen jedoch in Gegenwart von unter den Reaktionsbedingungen inerten Lösungsmitteln durchgeführt. Als solche eignen sich vorzugsweise polare aprotische Lösungsmittel, z. B. Säureamide wie Dimethylformamid, Dimethylacetamid und N-Methylpyrrolidon, weiterhin Dimethylsulfoxid, Nitrile wie Acetonitril oder Propionitril, Ketone wie Aceton, Methyläthylketon oder Methylisobutylketon, Äther wie Tetrahydrofuran oder Dioxan und aromatische und aliphatische Kohlenwasserstoffe wie Chlorbenzol oder Cyclohexan.

Als basische Verbindungen eignen sich die üblichen organischen Basen, z. B. tertiäre Amine wie Triäthylamin, und insbesondere anorganische Basen wie Alkali-Carbonate ($Na_2CO_3$, $K_2CO_3$). Sie werden in mindestens stöchiometrischen, vorzugsweise in mindestens äquimolaren Menten (bezogen auf II) bzw. in einem Überschuß von 5 bis 20 % eingesetzt.

Zur Durchführung des Verfahrens erwärmt man zweckmäßigerweise die Verbindung der Formel II mit der basischen Verbindung in einem geeigneten Lösungsmittel zunächst auf Reaktionstemperatur, wobei zumindest teilweise das Salz der Verbindung II gebildet wird. Dann gibt man die heterocyclische Komponente der Formel III, gegebenenfalls gelöst in dem gleichen Lösungsmittel, zu und rührt so lange bei erhöhter Temperatur, bis die Umsetzung beendet ist. Jedoch können die Reaktionspartner auch in anderer Reihenfolge zusammengegeben werden.

Die Reaktionstemperaturen sind in weiten Grenzen variierbar und liegen normalerweise zwischen 20 und 160 °C, vorzugsweise zwischen 40 und 85 °C, gegebenenfalls beim Siedepunkt des verwendeten Lösungsmittels. Die Reaktionsdauer hängt von der Temperatur, vom verwendeten Lösungsmittel und von dem Verhältnis II : III ab und wird geringer mit zunehmender Temperatur und wachsendem Überschuß an II. Gewöhnlich ist die Umsetzung nach 20 Minuten bis 8 Stunden beendet. Die Reaktion wird zweckmäßigerweise, jedoch nicht notwendigerweise unter Schutzgas durchgeführt. Als Inertgas wird Stickstoff bevorzugt.

Nach beendeter Reaktion wird von gebildetem Salz abfiltriert und das Lösungsmittel abdestilliert. Ein eventuell vorhandener Überschuß an II wird durch basische Extraktion entfernt.

Die erhaltenen Verbindungen der Formel I können durch Destillieren oder Chromatographieren weiter gereinigt werden, doch ist dies wegen der hohen Reinheit der Produkte im allgemeinen nicht nötig.

Die nachfolgenden Beispiele erläutern das erfindungsgemäße Verfahren.

## Herstellungsbeispiele

### Beispiel 1

2-[4'-(6"-Chlorbenzthiazol-2"-yl-oxi)-phenoxi]-propionsäureäthylester

21 g (0,1 Mol) 2-(4'-Hydroxyphenoxy)-propionsäureäthylester werden mit 16,6 g (0,12 Mol) Kaliumcarbonat in 150 ml Acetonitril anderthalb Stunden am Rückfluß erhitzt. Man gibt 24,75 g (0,1 Mol) 6-Chlor-2-methylsulfonylbenzthiazol in 100 ml Acetonitril gelöst zu und erhitzt weitere 5 Stunden zum Sieden, filtriert heiß vom Salzanteil ab, wäscht mit Acetonitril nach und destilliert das Lösungsmittel ab. Der verbleibende Rückstand wird in 200 ml Toluol gelöst, die Lösung mehrfach mit verdünnter $NaHCO_3$-Lösung gewaschen und über $Na_2SO_4$ getrocknet. Nach dem Abdestillieren des Toluols hinterbleiben 37 g (98 % d. Th.) an 2-[4'-(6"-Chlorbenzthiazol-2"-yl-oxy)-phenoxy]-propionsäureäthylester, Fp. 54 °C.

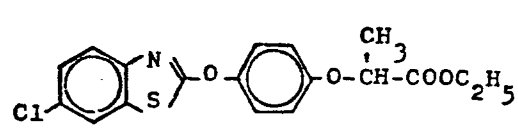

Der Salzrückstand (Gewicht nach dem Trocknen : 21,85 g) wird mehrfach mit heißem Ethanol extrahiert. Nach dem Eindampfen der Extrakte hinterbleiben 11,3 g (97 % d. Th.) Kaliummethylsulfinat.

In analoger Weise erhält man :

### Beispiel 2

Unter Verwendung von 31 g (0,1 g Mol) 2-Phenylsulfonylbenzthiazol nach 6 Stunden Reaktionszeit 32,2 g (94 % d. Th. 2-[4'-(Benzthiazol-2"-yl-oxy)-phenoxy]-propionsäureäthylester vom Kp. 201-204 °C/0.13 mbar :

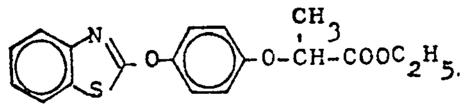

Beispiel 3

Unter Verwendung von 21 g (0,1 Mol) D-(+)-2-(4'-Hydroxyphenoxy)-propionsäureäthylester und 26,2 g (0,1 Mol) 6-Chlor-2-äthylsulfonylbenzthiazol nach 6 Stunden Reaktionszeit 35,5 g (94 % d. Th.) D-(+)-2-[4-(6-Chlorbenzthiazol-2-yl-oxy)-phenoxy]-propionsäureäthylester vom Fp. 53 °C und $[\alpha]_D^{20} = 30,2°$ (c = 1 m, Chloroform) :

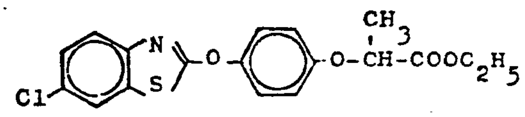

**Anspruch**

Verfahren zur Herstellung von 2-[4'-Benzthiazolyl-2''-oxi)-phenoxi] alkancarbonsäurederivaten der allgemeinen Formel I

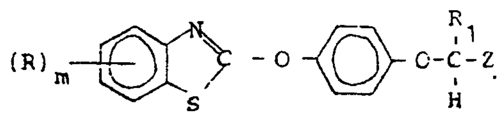

(I)

worin

R Halogen, $CF_3$, $NO_2$, CN, $(C_1-C_4)$-Alkyl, $(C_1-C_4)$-Alkoxi oder $(C_1-C_4)$-Alkylthio,

$R_1$ H oder $(C_1-C_4)$-Alkyl,

Z eine Gruppe der Formel

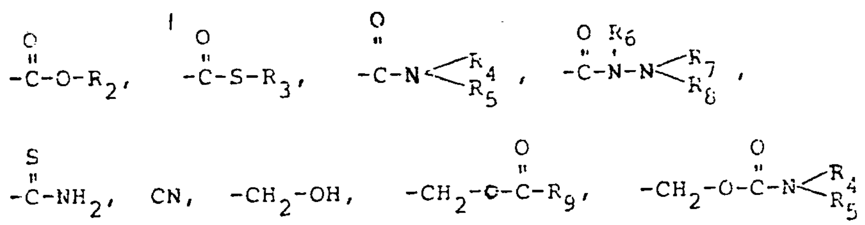

oder $-CH_2-O-SO_2-R_{10}$,

m 0 bis 2

$R_2$ H, $(C_1-C_{12})$-Alkyl, das gegebenenfalls durch 1-6 Halogen und/oder durch OH, $(C_1-C_6)$-Alkoxi, $(C_1-C_4)$-Alkylthio, $(C_1-C_6)$-Alkoxi-$(C_2-C_6)$-alkoxi, Halogen $(C_1-C_2)$-alkoxi, Methoxi-äthoxi-äthoxi, $(C_1-C_4)$-Alkylamino, Di-$(C_1-C_4)$-alkylamino, Phenyl, Oxiranyl und/oder Phenoxi substituiert ist, wobei letzteres ebenfalls ein- bis zweifach durch Halogen oder $(C_1-C_4)$-Alkyl substituiert sein kann,

$(C_5-C_6)$-Cycloalkyl, das gegebenenfalls durch Halogen oder Methyl substituiert ist, $(C_3-C_6)$-Alkenyl, Halogen-$(C_3-C_6)$-alkenyl oder $(C_5-C_6)$-Cycloalkenyl, $(C_3-C_4)$-Alkinyl, das gegebenenfalls ein- oder zweifach durch $(C_1-C_6)$-Alkyl, Phenyl, Halogen bzw. $(C_1-C_2)$-Alkoxi substituiert ist, Phenyl, das gegebenenfalls ein- bis dreifach durch $(C_1-C_4)$-Alkyl, $(C_1-C_4)$-Alkoxi, Halogen, $NO_2$ oder $CF_3$ substituiert ist, Furfuryl, Tetrahydrofurfuryl oder ein Kationäquivalent einer organischen oder anorganischen Base, oder Reste der Formeln

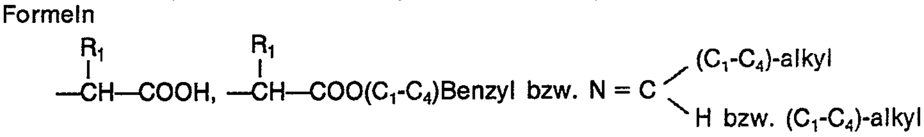

$R_3$ $(C_1-C_6)$-Alkyl, Phenyl-$(C_1-C_2)$-alkyl, wobei der Phenylrest ein- oder zweifach durch $(C_1-C_4)$-Alkyl und/oder Halogen substituiert sein kann, $(C_3-C_6)$-Alkenyl oder Phenyl, das auch ein- oder zweifach durch $(C_1-C_4)$-Alkyl und/oder Halogen substituiert sein kann,

$R_4$ und $R_5$ gleich oder verschieden sind und H, $(C_1-C_6)$-Alkyl, Hydroxy-$(C_1-C_6)$-alkyl, $(C_5-C_6)$-Cycloalkyl oder Phenyl, das gegebenenfalls ein- bis dreifach durch $(C_1-C_4)$-Alkyl, $(C_1-C_4)$-Alkoxi, Halogen oder $CF_3$ substituiert ist, mit der Maßgabe, daß $R_4$ und $R_5$ nicht gemeinsam Phenyl sind, bedeuten oder gemeinsam eine Methylenkette mit 2, 4 oder 5 Gliedern bilden, in der eine $CH_2$-Gruppe gegebenenfalls durch O, NH oder $N(CH_3)$ ersetzt sein kann,

$R_6$ H oder $CH_3$

$R_7$ H, $CH_3$ oder $C_2H_5$,

$R_8$ H, $CH_3$, $C_2H_5$- oder Phenyl,

$R_9$ ($C_1$-$C_6$)-Alkyl, das gegebenenfalls ein- bis dreifach durch Halogen substituiert ist, Cyclopropyl, ($C_3$-$C_6$)-Alkenyl, Phenyl, ($C_1$-$C_4$)-Alkyl-phenyl, ($C_1$-$C_4$)-Alkoxi-phenyl, Halogen-phenyl, Trifluormethyl-phenyl oder Nitrophenyl sowie

$R_{10}$ ($C_1$-$C_4$)-Alkyl oder Phenyl, das auch ein- oder zweifach durch Halogen, $CF_3$, $NO_2$ oder ($C_1$-$C_4$)-Alkyl substituiert sein kann,

bedeuten, dadurch gekennzeichnet, daß man Phenole der Formel II

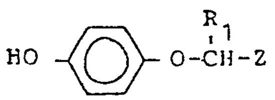

(II)

mit Verbindungen der Formel III

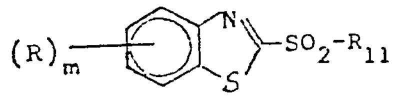

(III)

in der

$R_{11}$ einen niedermolekularen Alkylrest oder einen Phenylrest bedeudet, in Gegenwart basischer Verbindungen umsetzt.

**Claim**

A process for the manufacture of 2-[4'-Benzothiazolyl-2''-oxy)-phenoxy]-alkanecarboxylic acid derivatives of the general formula I

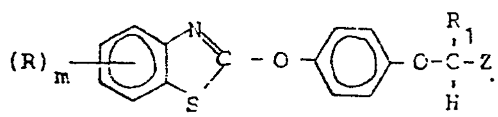

(I)

in which

R is halogen, $CF_3$, $NO_2$, CN ; alkyl, alkoxy or alkylthio each having from 1 to 4 carbon atoms ;

$R_1$ is H or ($C_1$-$C_4$)-alkyl,

Z is a group of the formulae

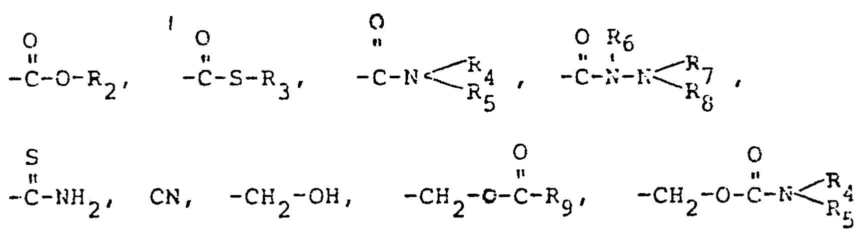

or $-CH_2-O-SO_2-R_{10}$,

m is an integer of from 0 to 2

$R_2$ is H, ($C_1$-$C_{12}$)-alkyl optionally substituted with 1 to 6 halogen atoms, and/or OH, ($C_1$-$C_6$)-alkoxy, ($C_1$-$C_4$)-alkylthio, ($C_1$-$C_6$)-alkoxy-($C_2$-$C_6$)-alkoxy, halo-($C_1$-$C_2$)-alkoxy, methoxyethoxyethoxy, ($C_1$-$C_4$)-alkylamino, di-($C_1$-$C_4$)-alkylamino, phenyl, oxiranyl and/or phenoxy, the latter one optionally mono- or disubstituted with halogen or ($C_1$-$C_4$)-alkyl ; ($C_5$-$C_6$)-cycloalkyl optionally substituted with halogen or methyl ; ($C_3$-$C_6$)-alkenyl, halo-($C_3$-$C_6$)-alkenyl or ($C_5$-$C_6$)-cycloalkenyl ; ($C_3$-$C_4$)-alkinyl optionally mono- or disubstituted with ($C_1$-$C_6$)-alkyl, phenyl, halogen or ($C_1$-$C_2$)-alkoxy ; phenyl optionally mono- to trisubstituted with ($C_1$-$C_4$)-alkyl or -alkoxy, halogen, $NO_2$ or $CF_3$ ; furfuryl, tetrahydrofurfuryl, a cation equivalent of an organic or inorganic base, or radicals of the formulae

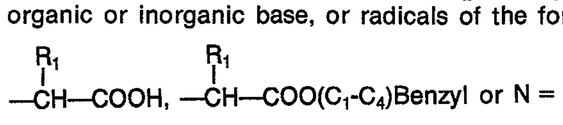

or

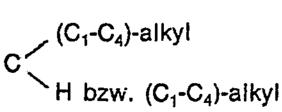

$R_3$ is ($C_1$-$C_6$)-alkyl, phenyl-($C_1$-$C_2$)-alkyl, the phenyl radical optionally mono- or disubstituted with ($C_1$-$C_4$)-alkyl and/or halogen ; ($C_3$-$C_6$)-alkenyl or phenyl optionally mono- or disubstituted with ($C_1$-$C_4$)-alkyl and/or halogen ;

$R_4$ and $R_5$, being identical or different, each are H, ($C_1$-$C_6$)-alkyl, hydroxy-($C_1$-$C_6$)-alkyl, ($C_5$-$C_6$)-cycloalkyl or phenyl optionally mono- to trisubstituted with ($C_1$-$C_4$)-alkyl or -alkoxy, halogen or $CF_3$ ; with the proviso that $R_4$ and $R_5$ together are not phenyl ; or together form a methylene chain having 2, 4 or 5 members, where one $CH_2$ group can be substituted by O, NH or $N(CH_3)$ ;

$R_6$ is H or $CH_3$ ;

$R_7$ is H, $CH_3$ or $C_2H_5$ ;

$R_8$ is H, $CH_3$, $C_2H_5$ or phenyl ;

$R_9$ is ($C_1$-$C_6$)-alkyl optionally mono- to trisubstituted with halogen, cyclopropyl, ($C_3$-$C_6$)-alkenyl, phenyl, ($C_1$-$C_4$)-alkylphenyl or -alkoxyphenyl, halophenyl, trifluoromethylphenyl or nitrophenyl ; and

$R_{10}$ is ($C_1$-$C_4$)-alkyl or phenyl optionally mono- or disubstituted with halogen, $CF_3$, $NO_2$ or ($C_1$-$C_4$)-alkyl ; which comprises reacting phenols of the formula II

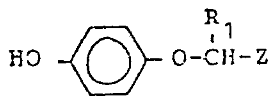

(II)

with compounds of the formula III

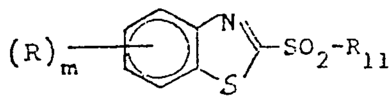

(III)

in which

$R_{11}$ is a lower alkyl or phenyl radical, in the presence of basic compounds.

**Revendication**

Procédé de préparation de dérivés d'acides (benzothiazolyl-2 oxy)-4 phénoxy-2 alcanoïques répondant à la formule I

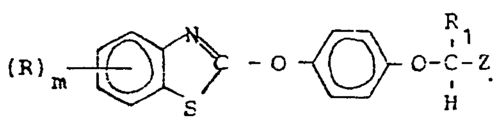

(I)

dans laquelle

R représente un halogène, $CF_3$, $NO_2$, CN, un alkyle en $C_1$-$C_4$, un alcoxy en $C_1$-$C_4$ ou un alkylthio en $C_1$-$C_4$,

$R_1$ représente l'hydrogène ou un alkyle en $C_1$-$C_4$,

Z représente un radical répondant à l'une des formules suivantes

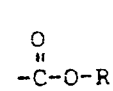 , 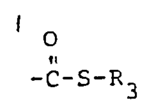 , 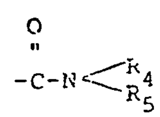 , 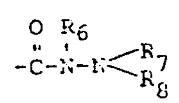 ,

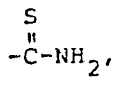 , CN, $-CH_2-OH$, 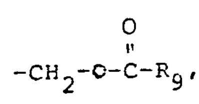 , 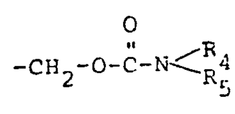

ou $-CH_2-O-SO_2-R_{10}$,

m désigne un nombre de 0 à 2,

$R_2$ représente : un atome d'hydrogène, un alkyle en $C_1$-$C_{12}$ qui porte éventuellement de 1 à 6 atomes d'halogènes et/ou un OH, un alcoxy en $C_1$-$C_6$, un alkylthio en $C_1$-$C_4$, un ($C_1$-$C_6$) alcoxy-($C_2$-$C_6$) alcoxy, un halogéno-alcoxy en $C_1$ ou $C_2$, un méthoxy-éthoxy-éthoxy, un alkylamino en $C_1$-$C_4$, un di-($C_1$-$C_4$) alkylamino, un phényle, un oxirannyle et/ou un phénoxy, ce dernier pouvant lui-même porter un ou deux substituants pris dans l'ensemble constitué par les halogènes et les alkyles en $C_1$-$C_4$, un cycloalkyle en $C_5$ ou $C_6$ éventuellement porteur d'un halogène ou d'un méthyle, un alcényle en $C_3$-$C_6$, un halogéno-

alcényle en $C_3$-$C_6$ ou un cyclo-alcényle en $C_5$ ou $C_6$, un alcynyle en $C_3$ ou $C_4$ qui porte éventuellement un ou deux substituants pris dans l'ensemble constitué par les alkyles en $C_1$-$C_6$, le phényle, les halogènes et les alcoxy en $C_1$ et $C_2$, un phényle éventuellement porteur d'un à trois substituants pris dans l'ensemble constitué par les alkyles en $C_1$-$C_4$, les alcoxy en $C_1$-$C_4$, les halogènes, $NO_2$ et $CF_3$, un furfuryle, un tétrahydrofurfuryle ou un équivalent cationique d'une base minérale ou organique, ou un radical répondant à l'une des formules suivantes

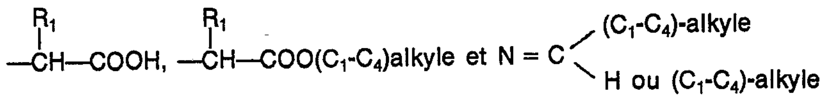

$R_3$ représente un alkyle en $C_1$-$C_6$, un phénylalkyle dont l'alkyle contient 1 ou 2 atomes de carbone et dont le phényle peut porter un ou deux substituants pris dans l'ensemble constitué par les alkyles en $C_1$-$C_4$ et/ou les halogènes, un alcényle en $C_3$-$C_6$, ou un phényle éventuellement porteur d'un ou deux substituants pris dans l'ensemble constitué par les alkyles en $C_1$-$C_4$ et/ou les halogènes,

$R_4$ et $R_5$ représentent chacun, indépendamment l'un de l'autre, l'hydrogène, un alkyle en $C_1$-$C_6$, un hydroxy-alkyle en $C_1$-$C_6$, un cycloalkyle en $C_5$ ou $C_6$ ou un phényle, lequel porte éventuellement de 1 à 3 substituants pris dans l'ensemble constitué par les alkyles en $C_1$-$C_4$, les alcoxy en $C_1$-$C_4$, les halogènes et $CF_3$, avec la condition que $R_4$ et $R_5$ ne représentent pas ensemble un radical phényle, ou encore $R_4$ et $R_5$ forment ensemble une chaîne méthylénique contenant 2, 4 ou 5 maillons dans laquelle l'un des radicaux $CH_2$ peut être remplacé par O, NH ou $N(CH_3)$,

$R_6$ représente H ou $CH_3$,

$R_7$ représente H, $CH_3$ ou $C_2H_5$,

$R_8$ représente H, $CH_3$, $C_2H_5$ ou un phényle,

$R_9$ représente un alkyle en $C_1$-$C_6$ éventuellement porteur d'1 à 3 atomes d'halogène, un cyclopropyle, un alcényle en $C_3$-$C_6$, un phényle, un alkylphényle dont l'alkyle contient de 1 à 4 atomes de carbone, un alcoxyphényle dont l'alcoxy contient de 1 à 4 atomes de carbone, un halogénophényle, un trifluorométhyl-phényle ou un nitrophényle, et

$R_{10}$ représente un alkyle en $C_1$-$C_4$ ou un phényle, lequel peut porter un ou deux substituants pris dans l'ensemble constitué par les halogènes, $CF_3$, $NO_2$ et les alkyles en $C_1$-$C_4$,

procédé caractérisé en ce qu'on fait réagir des phénols de formule II

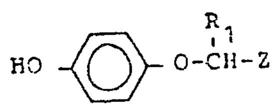

(II)

avec des composés répondant à la formule III

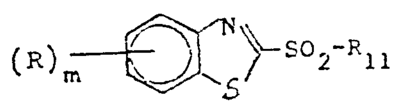

(III)

dans laquelle $R_{11}$ représente un radical alkyle à bas poids moléculaire ou un radical phényle, en présence de composés basiques.